# EUROPÄISCHE PATENTSCHRIFT

(11) **EP 1 292 268 B1**
(45) Veröffentlichungstag und Bekanntmachung des Hinweises auf die Patenterteilung: **23.02.2005**
(21) Anmeldenummer: 01943502.3
(22) Anmeldetag: 13.06.2001
(51) Int. Cl.: A61K 7/13

(54) **MITTEL ZUM FÄRBEN VON KERATINHALTIGEN FASERN**
AGENT FOR DYEING KERATIN CONTAINING FIBERS
AGENT DE COLORATION DE FIBRES KERATINIQUES

(30) Priorität: 21.06.2000 DE 10029384
(43) Veröffentlichungstag der Anmeldung: 19.03.2003
(73) Patentinhaber: Henkel Kommanditgesellschaft auf Aktien, 40589 Düsseldorf-Holthausen (DE)
(72) Erfinder: OBERKOBUSCH, Doris, 40591 Düsseldorf (DE); HÖFFKES, Horst, 40595 Düsseldorf (DE); MÖLLER, Hinrich, 40789 Monheim (DE); GROSS, Wibke, 40549 Düsseldorf (DE); MARTIN, Hans-Dieter, 40591 Düsseldorf (DE)
(86) Internationale Anmeldenummer: PCT/EP2001/006690
(87) Internationale Veröffentlichungsnummer: WO 2001/097764

(56) Entgegenhaltungen:
- US-A- 5 101 032

## Beschreibung

Die Erfindung betrifft ein Mittel zum Färben von keratinhaltigen Fasern, insbesondere menschlichen Haaren, das mindestens ein Cyclohexenonderivat enthält, die Verwendung dieser Verbindungen in Mitteln zum Färben von keratinhaitigen Fasern sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstorfe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminopheriol, N,N-Bis-(2-hydroxyethy))-p-phenyiendiamin, 2-(2,5-Diaminophenyl)-ethanol, 2-(2,5-Diaminophenoxy)-ethanol, 1-Phenyt-3-carboxysmido-4-amino-pyrazolon-5, 4-Amino-3-methylphenol, 2-Aminomethyl-4-aminophenol, 2-Hydroxyrnethyl-4-aminophenol, 2-Hydroxy-4,5,6-triaminopyrianidin, 2,4-Dihydroxy-5,6-diaminopyrimidin und 2,5,6-Triamino-4-hydroxypyrimidin.

Als Kuppierkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, dass die Färbungen häufig nur über unzureichende Echtheitseigenschaften verfügen.

Die Verwendung der unten näher beschriebenen Cyclohexenonderivate zum Färben von keratinhaltigen Fasern ist bislang nicht bekannt.

Aufgabe der vorliegenden Erfindung ist es, Färbemittel für Keratinfasem, insbesondere menschliche Haare, bereitzustellen, die hinsichtlich der Farbtiefe, derGrauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, dass die in der Formel I dargestellten Cyclohexenonderivate sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend mindestens ein Cyclohexenonderivat mit der Formel I, 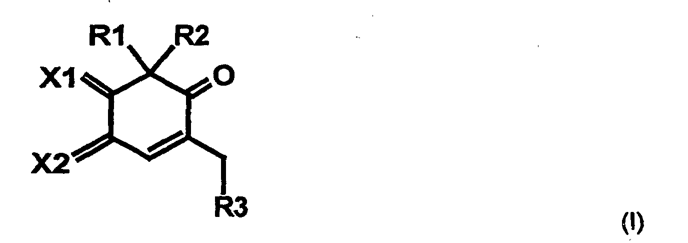 in der bedeuten
in der R¹, R², R³ unabhängig voneinander ein Wasserstoffatom oder eine C₁-C₄-Alkylgruppe, wobei R¹ und R² zusammen auch einen Ring bilden können,
X¹ und X² ein Sauerstoff-, ein Schwefelatom oder gemeinsam eine 1,2-Arylendiiminogruppe, die ihrerseits im Aromaten durch Halogenatome, C₁-C₄-Alkyl-, C₁-C₄-Alkoxy-, Nitro-, Amino-, C₁-C₄-Alkylamino-, Hydroxy-, Carboxy-, Sulfogruppen oder einen weiteren kondensierten aromatischen Ring substituiert und an einem der Stickstoffatome durch eine C₁-C₆-Alkyl-, Aralkyl-, Aryl-, C₂-C₄-Alkenyl- oder C₁-C₈-Hydroxyalkyl-, Carboxyalkylgruppe quaterniert sein kann, wobei der weitere kondensierte aromatische Ring durch Halogenatome, C₁-C₄-Alkyl-, C₁-C₄-Alkoxy-, Nitro-, Amino-, C₁-C₄-Alkylamino-, Hydroxy-, Carboxy-, Sulfogruppen substituiert sein kann,
oder dessen tautomere Formen oder dessen physiologisch verträgliche Salze.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyloder Acetylcellulose und synthetischer Fasern, wie z. B. Polyamid-, Polyacrylnitril-, Polyurethan- und Polyesterfasern verwendet werden.

Die Verbindungen mit der Formel I sind zum großen Teil literaturbekannt, im Handel erhältlich oder nach bekannten Syntheseverfahren herstellbar.

Vorzugsweise sind die Verbindungen mit der Formel I ausgewählt aus 3,3,5-Trimethylcyclohex-5-en-1,2,4-trion, 3,5-Dibutyl-2-hydroxy-2,5-cyclohexadien-1,4-dion, 2-Ethyl-5-hydroxy-2,5-cyclohexadien-1,4-dion, 2-Hydroxy-3-methylphenazin, 3,10-Dimethyl-2(10H)-phenazinon, 3-Methyl-2,8-phenazindiol, 3,7-Dihydroxy-2,5-dimethyl-phenaziniummethosulfat, 1,7-Dimethyl-2,8-phenazindiol, 3,7-Dihydroxy-2,8-dimethyl-5-phenyl-phenazinium-sulfate, 1,1,3,5-Tetramethyl-2-oxo-1,2-dihydrophenazinium-, 1,1,3,5-Tetramethyl-7,8-dimethoxy-1,2-dihydro-2-oxophenazinium-tetrafluorborat 1,1,3,5-Tetramethyl-2-oxo-8-nitro-1,2-dihydrophenazinium-tetrafluorborat, 1,1,3-Trimethyl-2-oxo-8-nitro-1,2-dihydrophenazinium-, 1,1,3-Trimethyl-5-phenyl-1,2-dihydro-2-oxophenaziniumtetrafluoroborat, 8-Chlor-1,1,3,5-tetramethyl-2-oxo-1,2-dihydrophenazinium-, 8-Chlor-5-isopropyl-1,1,3-trimethyl-2-oxo-1,2-dihydrophenazinium-tetrafluorborat, -p-toluolsulfonat, -iodid, -bromid, -chlorid, -sulfat, -methylsulfat, -tetrachlorzinkat, -perchlorat, -perfluormethansulfonat, -methansulfonat, -benzolsulfonat, sowie deren beliebigen Gemischen.

Die voranstehend genannten Cyclohexenonderivate mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Verbindungen der Formel I dimerisieren in Gegenwart von Luftsauerstoff zu farbigen Verbindungen, die als direktziehende Färbemittel eingesetzt werden können und sich durch gute Echtheiten auszeichnen.

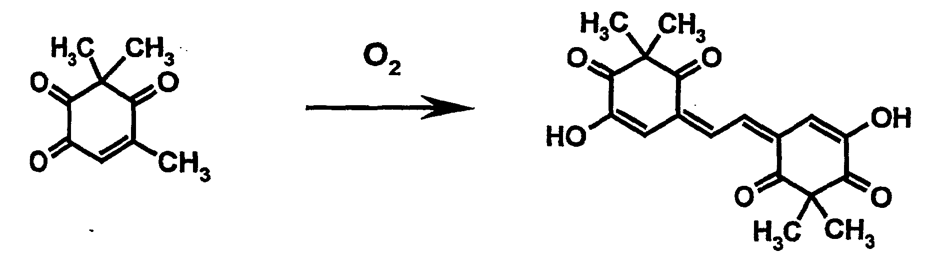

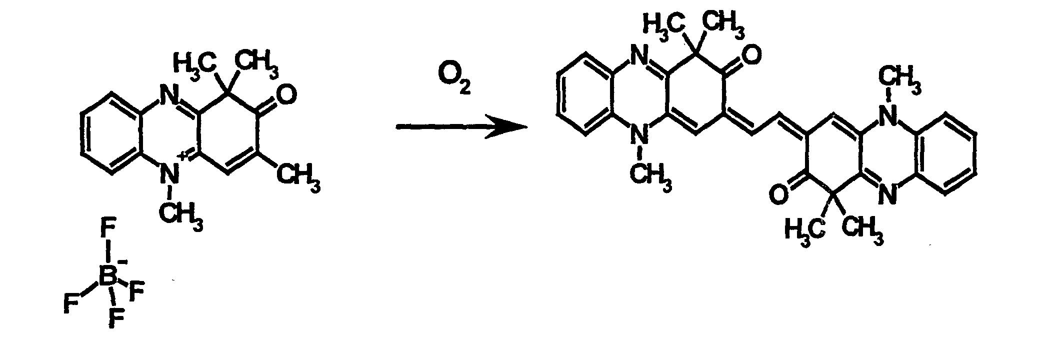

Zur Erweiterung des Farbspektrums können die Verbindungen vor, während oder nach der Dimerisierung mit Aminen umgesetzt werden. Ferner ist vor der Dimerisierung aufgrund der C-H-Acidität des Monomers auch eine Umsetzung mit Aldehyden möglich.

Färbemittel, die als färbende Komponente Cyclohexenonderivate mit der Formel I allein enthalten, werden bevorzugt für hellere Färbungen eingesetzt. Färbungen mit erhöhter Brillanz und verbesserten Echtheitseigenschaften (Lichtechtheit, Waschechtheit, Reibechtheit) über einen weiten Nuancenbereich werden erzielt, wenn die erfindungsgemäß eingesetzten Verbindungen mit der Formel I mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder aromatischen Aldehyden, verwendet werden. Dies sind einerseits Verbindungen, die für sich alleine keratinhaltige Fasern nur schwach färben und erst gemeinsam mit den Verbindungen der Formel 1 brillante Färbungen ergeben. Andererseits sind darunter aber auch Verbindungen, die bereits als Oxidationsfarbstoffvorprodukte eingesetzt werden.

In allen Färbemitteln können auch mehrere verschiedene Cyclohexenonderivate der Formel I gemeinsam zum Einsatz kommen; ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von Cyclohexenonderivaten der Formel I mit den genannten Verbindungen der Komponenten B darstellen, als direktziehende Färbemittel. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Geeignete Verbindungen mit primärer oder sekundärer Aminogruppe als Komponente B sind z. B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilina-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, -phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4 Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogaltol, 3,5-Diamino-4-hydroxybrenzcatechin, 1,4-Bis-(4-aminophenyl)-1,4-diazacycloheptan, Bis-(5-amino-2-hydroxyphenyl)-methan, aromatische Nitrile, Nitrogruppen-haltige Aminoverbindungen, wie 3-Amino-6-methylamino-2-nitro-pyridin, Pikraminsäure, 1-(2(3)-Nitro-4-amino)-phenylazo-2-hydroxy-7-trimethylammoniumnaphthalin-chlorid, 1-Hydroxy-2-amino-4,6-dinitro-benzol, 1-Amino-2-nitro-4-bis-(2-hydroxyethyl)-amino-benzol, 1-Amino-2-(2-hydroxyethyl)-amino-5-nitrobenzol (HC Yellow Nr. 5), 1-Amino-2-nitro-4-(2-hydroxyethyl)-aminobenzol (HC Red Nr. 7), 2-Chloro-5-nitro-N-hydroxyethyl-1,4-phenylendiamin, 1-(2-Hydroxyethyl)-amino-2-nitro-4-amino-benzol (HC Red Nr. 3), 4-Amino-3-nitrophenol, 4-Amino-2-nitrophenol, 6-Nitro-o-toluidin, 1-Amino-3-methyl-4-(2-hydroxyethyl)-amino-6-nitrobenzol (HC Violet Nr. 1), 1-Amino-2-nitro-4-(2,3-dihydroxypropyl)-amino-5-chlorobenzol (HC Red Nr. 10), 4-Amino-2-nitrodiphenyl-amino-2'-carbonsäure (2-(4-Amino-2-nitroanifino)-benzoesäure), 6-Nitro-2,5-cfiaminopyridin, 2 Amino-6-chloro-4-nitrophenol, 2 Amino-6-chloro-4-nitrophenol, 1-Amino-2-(3-nitrophenylazo)-7-phenylazo-8-naphthol-3,6-disulfonsäure, Dinatriumsalz (Acid blue Nr. 29), 1-Amino-2-(2-hydroxy-4-nitrophenylazo)-8-naphthol-3,6-disulfonsäure, Dinatriumsalz (Palatinchrome green), 1-Amino-2-(3-chlor-2-hydroxy-5-nitrophenylazo)-8-naphthol-3,6-disulfonsäure, Dinatriumsalz (Gallion), 4-Amino-4'-nitrostilben-2,2'disulfonsäure, Dinatriumsalz, 2,4-Diamino-3',5'-dinitro-2'-hydroxy-5-methyl-azobenzol (Mordant brown 4), 4'-Amino-4-nitrodiphenyfamin-2-sulfonsäure, 4' Amino-3'-nitrobenzophenon-2-carbonsäure, 1-Amino-4-nitro-2-(2-nitrobenzylidenamino)-benzol, 2-[2-(Diethylamirro)-ethylamino]-5-nitroanitin, 3-Amino-4-hydroxy-5-nitrobenzolsulfonsäure, (Hydrat), 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methy)-6-nitrobenzo), 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-niftodiphenylamin-2'-carbonsäure, 2-Amino-6-chlor-4-nitropheanol, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 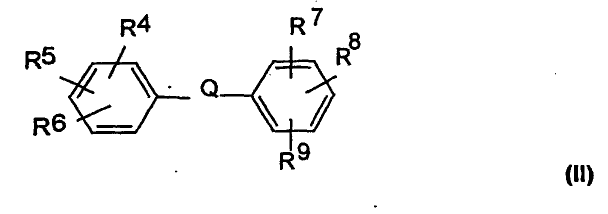 in der R⁴ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht, R⁵, R⁶, R⁷, R⁸ und R⁹ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄alkylgruppen substituiert sein kann, stehen, und
Q für eine direkte Bindung, eine gesättigte oder ungesättigte, ggf. durchHydroxygruppen substituierte Kohlenstoffkette mit 1 bis 4 Kohlenstoffatomen, eine Carbonyl-, Sulfonyloder Iminogruppe, ein Sauerstoff- oder Schwefelatom, oder eine Gruppe mit der Formel III

Z-(CH₂-Y-CH₂Z')ₒ (III) (III)

in der Y eine direkte Bindung, eine CH₂- oder CHOH-Gruppe bedeutet,
Z und Z' unabhängig voneinander für ein Sauerstoffatom, eine NR¹⁰-Gruppe, worin
R¹⁰ Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und
o eine Zahl von 1 bis 4 bedeutet,
wie beispielsweise 4,4'-Diaminostilben und dessen Hydrochlorid, 4,4'-Diaminostitben-2,2'disulfonsäure-mono- oder -di-Na-Salz, 4-Amino-4'-dimethylaminostilben und dessen Hydrochlorid, 4,4-Diaminodiphenylmethan, -sulfid, -sulfoxid, -amin, .4,4'-Diaminodiphenylamin-2-sulfonsäure, 4,4'-Diaminobenzophenon, -diphenylether, 3,3',4,4'-Tetraaininodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 4,5-Diamino-1-(2-hydroxyethyl)-pyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indol- und Indolinderivaten, wie 4-, 5-, 6-, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Satz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als Aminosäuren kommen bevorzugt alle natürlich vorkommenden und synthetischen α-Aminosäuren in Frage, die z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren erhalten werden können. Dabei können sowohl sauer als auch alkalisch reagierende Aminosäuren eingesetzt werden. Bevorzugte Aminosäuren sind Arginin, β-Alanin, Histidin, Tyrosin, Phenylalanin, DOPA (Dihydroxyphenylalanin), Ornithin, Lysin und Tryptophan, Prolin. Aber auch andere Aminosäuren, wie z.B. 6-Aminocapronsäure, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Als geeignete Beispiele für aromatische Aldehyde können z. B. Salicylaldehyd, 3-Hydroxybenzaldehyd, 4-Hydroxybenzaldehyd, o-Anisaldehyd, m-Anisaldehyd, p-Anisaldehyd, 4-Hydroxy-3-methylbenzaldehyd, 2-Hydroxy-3-methylbenzaldehyd, 2-Hydroxy-5-methylbenzaldehyd, 2-Hydroxy-4-methylbenzaldehyd, 2,3-Dihydroxybenzaldehyd, 2,5-Dihydroxybenzaldehyd, 3,4- Dihydroxybenzaldehyd, 2,4- Dihydroxybenzaldehyd, 3,5-Dihydroxybenzaldehyd, 2,3-Dihydrobenzo[b]furan-5-carboxaldehyd, Piperonal, 4-Ethoxy-benzaldehyd, 3,5-Dimethyl-4-hydroxybenzaldehyd, Vanillin, Isovanillin, 2,3,4-Trihydroxybenzaldehyd, 2,4,5-Trihydroxybenzaldehyd, 2,4,6-Trihydroxybenzaldehyd, 3,4,5-Trihydroxybenzaldehyd, 3-Chlor-4-hydroxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,4-Dihydroxy-5-methoxybenzaldehyd, 2-Hydroxy-1-naphthaldehyd, 4-Hydroxy-1-naphthaldehyd, 1-Hydroxy-2-naphthaldehyd, 2-Methoxy-1-naphthaldehyd, 4-Methoxy-1-naphthaldehyd, 2-Hydroxyacetophenon, 4-Hydroxyacetophenon, 2,4-Dihydroxyacetophenon, 2-Hydroxyzimtaldehyd, 4-Hydroxyzimtaldehyd, 2,4-Dihydroxyzimtaldehyd, 4-Hydroxy-benzylidenaceton, 4-Hydroxy-3-methoxybenzylidenaceton, 4-Hydroxy-3-methoxyzimtaldehyd (Coniferylaldehyd), 3,5-Dimethoxy-4-hydroxyzimtaldehyd, 4-Hydroxycinnamylidenacetaldehyd, 4-Methoxycinnamylidenacetaldehyd genannt werden.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis-(2-hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Diethylaminomethyl-4-aminophenol, 2-Dimethylaminomethyl-4-aminophenol, 2,6-Dichlor 4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol und 5,6-Dihydroxyindolin, β-Alanin, L-Prolin, L-Lysin, DL-Tyrosin sowie deren mit vorzugsweise anorganischen Säuren gebildeten physiologisch verträglichen Salze.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden. Weiterhin kann es erfindungsgemäß besonders bevorzugt sein, die Verbindung der Formel I und die Verbindung der Komponente B im molaren Mengenverhältnis von 2 : 1 bis 1 : 2, insbesondere etwa äquimolar, einzusetzen. Beim Einsatz von Verbindungen der Komponente B, die mehrere Aminogruppen enthalten, werden die Verbindungen der Formel I einerseits und die Verbindungen der Komponente B andererseits bevorzugt in stöchiometrisch molaren Mengenverhältnissen eingesetzt.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis-(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhattigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykotethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylgiycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessiasäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykoletherdruppe oder eine, Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmittein verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mitquatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobornylacrylat-Copolymere, Methylvinylether/Maleinsäureanhydrid-Copolymere und Acrylsäure/Ethylacrylat/N-tert.-Butylacrylamid-Terpolymers,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den FärbemittelnAmmonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobeiNatriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung von Cyclohexenonderivaten mit der Formel I, 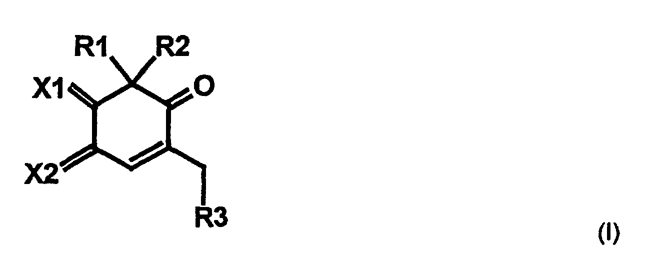 in der R¹,R²,R³,X¹ und X² wie oben definiert sind,
oder dessen tautomere Formen oder dessen physiologisch verträgliche Salze als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
A) mindestens ein Cyclohexenonderivat mit der Formel I, 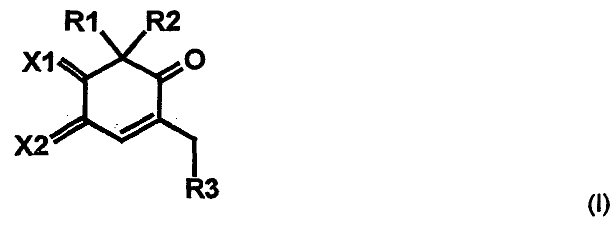 in der R¹, R², R³, X¹ und X² wie oben definiert sind, oder dessen tautomere Formen oder dessen physiologisch verträgliche Salze und
B) mindestens eine Verbindung ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten Oligopeptiden und/oder aromatischen Aldehyden,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Cyclohexenonderivate der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Cyclohexenonderivate der Formel 1 und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung der eingesetzten Färbekomponenten

### Beispiel 1: Darstellung von 3,3,5-Trimethyl-cyclohex-5-en-1,2,4-trion

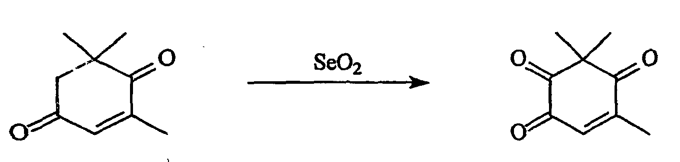

In einem 1 I Dreihalskolben mit KPG-Rührer und Rückflußkühler wurden 65 g (0,43 mol) käüfliches 6-Oxoisophoron (3,5,5-Trimethyl-cyclohex-2-en-1,4-dion) in 100 ml destilliertem 1,4-Dioxan mit einer Lösung von 50 g (0,45 mol) Selendioxid in 400 ml destilliertem 1,4-Diöxan versetzt. Das Reaktionsgemisch wurde rasch auf 130°C Ölbadtemperatur aufgeheizt, wobei sich die Lösung rot verfärbte. Nach ca. 7 Stunden wurde vom ausgefallenen Selen abfiltriert und das Lösungsmittel am Rotationsverdampfer abgezogen. Der Rückstand wurde in eine Kristallisationsschale gegossen, worin er erstarrte. Es wurde aus Hexan umkristallisiert. Das von den roten selenorganischen Verbindungen noch orange gefärbte Produkt wurde gemörsert und in eine Extraktionshülse gegeben. Anschließend wurde mit 700 ml Hexan extrahiert, bis das im Soxhletextraktor verbleibende Hexan nur noch schwach gelb gefärbt war. Das Lösungsmittel wurde am Rotationsverdampfer abgezogen und die Extraktion nochmals mit 700 ml Hexan wiederholt. Beim Erkalten kristallisierte das Produkt bereits in gelben Nadeln aus.
- Ausbeute:: 43 g (0,26 mol), 60 % der Theorie
- Smp.:: 87°C

### Beispiel 2: Darstellung von 1,1,3,5 Tetramethyl-1H-phenazin-2-on bzw. 1,1,3,5-Tetramethyl-5a,9a-dihydro-1H-phenazin-2-on

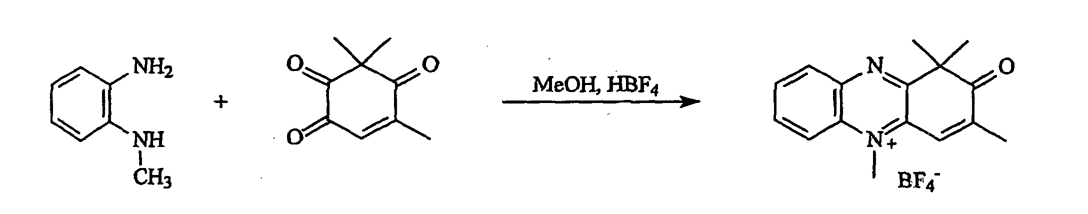

In einem 250 ml Kolben wurden 2,72 g (16 mmol) 3,3,5-Trimethyl-cyclohex-5-en-1,2,4-trion in 80 ml Methanol vorgelegt und mit 25 ml 32%iger Tetrafluorborsäure versetzt. Dann wurden 2,00 g (16 mmol) N-Methyl-o-phenylendiamin in 40 ml Methanol schnell zugetropft und das Reaktionsgemisch 8 Stunden bei Raumtemperatur gerührt. Es fiel ein gelb-beiger Feststoff aus, welcher abfiltriert wurde. Es wurde mehrmals mit kleinen Mengen Methanol gewaschen.

Ausbeute: 4,15 g (12,2 mmol), 75% der Theorie

### Herstellung der Färbegele

Die angegebene Menge der Komponente mit der Formel wurde ggf. unter Zusatz von Ammoniak in Wasser gelöst und die Lösung mit 2 % Natrosol®250 HR verdickt. (Färbegele 1 und 2) bzw. in eine Mischung aus Cetiol®868, Paraffinöl, Glycerinmonostearat und Dehydol®L24 eingetragen (Färbegel 3). Die Zusammensetzung der Farbgele ist in der folgenden Tabelle 1 wiedergegeben.

### Zusammensetzung der Färbegele (Tab. 1)

| -------- | Färbegel1 Menge (g) | Färbegel 2 Menge (g) | Färbegel 3 Menge (g) |
|---|---|---|---|
| 3,3,5-Trimethylcyclohex-5-en-1,2,4-trion | 3,3 | -------- | -------- |
| 1,1,3,5-Tetramethyl-2-oxo-1,2-dihydrophenazinium-tetrafluoroborat | -------- | 6,8 | -------- |
| 1,1,3-Trimethyl-5-phenyl-1,2-dihydro-2-oxophenaziniumtetrafluoroborat | -------- | -------- | 3,91 |
| Natrosol® 250 HR | 2 | 2 | -------- |
| Cetiol® 868 | -------- | -------- | 19,22 |
| Paraffinöl, dünnflüssig | -------- | -------- | 57,65 |
| Glycerinmonostearat | -------- | -------- | 9,61 |
| Dehydol®LS4 | -------- | -------- | 9,61 |
| Wasser, dest. | ad 100 | ad 100 | -------- |

### Herstellung der Farbcremes

Texapon®NSO, Dehyton®K, Hydrenol®D, Lorol® techn. und Eumulgin® B2 wurden unter Zusatz von ca. 10 g Wasser bei 80° C emulgiert. Separat wurden die Farbstoffe in Wasser gelöst, mit Ammoniak oder NaOH sowie Ascorbinsäure und Natriumsulfit versetzt und die Lösung mit der Emulsion vermischt. Die einzelnen Zusammensetzungen sind in Tabelle 2 wiedergegeben.

### Legende zu Tabellen 1 und 2

- Natrosol® 250 HR =: Hydroxyethylcellulose
- Texapon® NSO =: Na-Laurylethersulfat (26.5 - 27%ig)
- Dehyton® K =: Cocoamidopropylbetain (29 - 32%ig)
- Hydrenol® D =: Cetearylakohol
- Lorol®, techn. =: Kokosfettalkohol
- Eumulgin® B 2 =: Ceteareth-20

Das Färbegel 1 oder 2 wurde mit einer der Farbcremes aus Tab. 2 im Gewichtsverhältnis 1:1 vermischt, mit Ammoniak oder Weinsäure auf den in Tab. 3 angegebenen pH-Wert eingestellt und auf Haarsträhnen (Kerling, naturweiß) appliziert.Die Einwirkzeit betrug 30 min. bei 32° C.

Es wurden die in Tab. 3 genannten Farbergebnisse gemäß Deutschem Farbatlas erhalten:

**Tabelle 2**

| **Komponenten** | **A** | **B** | **C** | **D** | **E** | **F** |
|---|---|---|---|---|---|---|
| Texapon® NSO | 20,00 | 20,00 | 20,00 | 20,00 | 20,00 | 20,00 |
| Dehyton® K | 12,50 | 12,50 | 12,50 | 12,50 | 12,50 | 12,50 |
| Hydrenol® D | 8,50 | 8,50 | 8,50 | 8,50 | 8,50 | 8,50 |
| Lorol®, techn | 2,00 | 2,00 | 2,00 | 2,00 | 2,00 | 2,00 |
| Eumulgin® B2 | 0,75 | 0,75 | 0,75 | 0,75 | 0,75 | 0,75 |
| 4,4'-Diaminodiphenylendiamin*H₂SO₄ | 5,95 | - | - | - | - | - |
| N,N-Bis(2-hydroxyethyl)-p-phenylendiamin | - | 5,89 | - | - | - | - |
| 1-(2-Hydroxyethyl)-2,5-diaminobenzol | - | - | 5,01 | - | - | - |
| 2-Methylamino-3-amino-6-methoxypyridin | - | - | - | 4,52 | - | - |
| 4-(N,N-Dimethylamino)benzaldehyd | - | - | - | - | 3,00 | - |
| 4-Dimethylaminozimtaldehyd | - | - | - | - | - | 3,50 |
| Natriumsulfit | 0,10 | 0,10 | 0,10 | 0,10 | - | - |
| Ascorbinsäure | 0,10 | 0,10 | 0,10 | 0,10 | - | - |
| Ammoniak, 25%ig | 4,50 | 5,00 | 4,00 | 4,50 | 4,00 | 4,50 |
| Wasser, dest. | ad 100 | ad 100 | ad 100 | Ad 100 | ad 100 | ad 100 |
| **pH-Wert** | **8,95** | **9,40** | **8,78** | **9,08** | **9,27** | **8,89** |

| Färbegel | Farbcreme | pH | Farbergebnis |
|---|---|---|---|
| 1 | - | 2,3 | maisgelb |
| 2 | - | 6,1 | dunkelblau |
| 2 | - | 9,3 | blauschwarz |
| 1 | A | 8,85 | olivbraun |
| 1 | B | 8,9 | nußbraun |
| 1 | B | 6,2 | dunkelbraun |
| 1 | C | 9,5 | champagner |
| 1 | D | 9,3 | olivbraun |
| 2 | A | 5,6 | nickelgrün |
| 2 | A | 9,4 | dunkelgrün |
| 2 | B | 6,6 | dunkelbraun |
| 2 | B | 8,8 | grüngrau |
| 2 | C | 6,6 | dunkelgrün |
| 2 | C | 9,4 | dunkelgrün |
| 2 | D | 5,8 | oliv |
| 2 | D | 9,1 | olivbraun |
| 2 | E | 6,7 | dunkelblau |
| 2 | F | 5,9 | dunkelgrün |
| 3 | A | 9,5 | graugrün |
| 3 | B | 9,4 | nutria |
| 3 | C | 9,3 | haarbraun |
| 3 | D | 9,1 | graugelb |

## Patentansprüche

1. Mittel zum Färben von keratinhaltigen Fasern, insbesondere menschlichen Haaren, enthaltend als färbende Komponente mindestens ein Cyclohexenonderivat mit der Formel I, 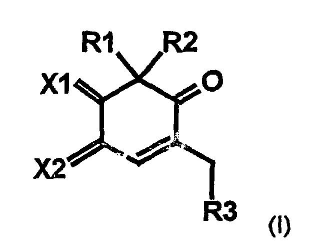 In der bedeuten
R¹, R², R³ unabhängig voneinander ein Wasserstoffatom oder eine C₁-C₄-Alkylgruppe, wobei R¹ und R² zusammen auch einen Ring bilden können,
X¹ und X² ein Sauerstoff-, ein Schwefelatorn oder gemeinsam eine 1,2-Arylendiiminogruppe, die ihrerseits im Aromaten durch Halogenatome, C₁-C₄-Alkyl-, C₁-C₄-Alkoxy-, Nitro-, Amino-, C₁-C₄-Alkylamino-, Hydroxy-, Carboxy-, Sulfogruppen oder einen weiteren kondensierten aromatischen Ring substituiert und an einem der Stickstoffatome durch eine C₁-C₆-Alkyl-, Aralkyl-, Aryl-, C₂-C₄-Alkenyl-, C₁-C₆-Hydroxyalkyl- oder Carboxyalkylgruppe quaterniert sein kann,
wobei der weitere kondensierte aromatische Ring durch Halogenatome, C₁-C₄-Alkyl-, C₁-C₄-Alkoxy-, Nitro-, Amino-, C₁-C₄-Alkylamino-, Hydroxy-, Carboxy-, Sulfogruppen substituiert sein kann,
oder dessen tautomere Formen oder dessen physiologisch verträgliche Salze.

2. Mittel nach Anspruch 1, **dadurch gekennzeichnet, dass** als Verbindungen mit der Formel I 3,3,5-Trimethylcyclohex-5-en-1,2,4-trion, 3,5-Dibutyl-2-hydroxy-2,5-cyclohexadien-1,4-dion, 2-Ethyl-5-hydroxy-2,5-cyclohexadien-1,4-dion, 2-Hydroxy-3-methylphenazin, 3,10-Dimethyl-2(10H)-phenazinon, 3-Methyl-2,8-phenazindiol, 3,7-Dihydroxy-2,5-dimethyl-phenazinium-methosulfat, 1,7-Dimethyl-2,8-phenazindiol, 3,7-Dihydroxy-2,8-dimethyl-5-phenyl-phenazinium-sulfate, 1,1,3,5-Tetramethyl-2-oxo-1,2-dihydrophenazinium-, 1,1,3,5-Tetramethyl-7,8-dimethoxy-1,2-dihydro-2-oxophenaninium-tetrafluoroborat, 1,1,3,5-Tetramethyl-2-oxo-8-nitro-1,2-dihydrophenazinium-tetrafluorborat, 1,1,3-Trimethyl-2-oxo-8-nitro-1,2-dihydrophenazinium-, 1,1,3-Trimethyl-5-phenyl-1,2-dihydro-2-oxophenazinium-tetrafluoroborat, 8-Chlor-1,1,3,5-tetramethy!-2-oxo-1,2-dihydrophenazinium-, 8-Chlor-5-isopropyl-1,1,3-trimethyl-2-oxo-1,2-dihydrophenazinium-tetrafluorborat, -p-toluolsulfonat, -iodid, -bromid, -chlorid, -sulfat, -methylsulfat, -tetrachlorzinkat, -perchlorat, -perfluormethansulfonat, -methansulfonat, -benzolsulfonat.sowie deren beliebigen Gemischen eingesetzt werden.

3. Mittel nach, einem der Ansprüche 1 bis 2, **dadurch gekennzeichnet, dass** die Cydohexenonderivate der Formel 1 in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, dass** es zusätzlich mindestens eine Verbindung, ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren, aus 2 bis 9 Aminosäuren aufgebauten-Oligopeptiden und/oder aromatischen Aldehyden, enthält.

5. Mittel nach Anspruch 4, **dadurch gekennzeichnet, dass** die weitere Verbindung ausgewählt ist aus
primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlorp-phenytendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminototuol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor , 4-Methytamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 2-Hydroxymethyl-4-aminophenol, 2-Diethylaminomethyl-4-aminophenol, 2-Dimethylaminomethyl-4-aminophenol, 2,6-Dichlor-4-aminophenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sutfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Traminophenol-trihydrochlorid, Pentaaminobenzolpentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogal(ol-dihydrochlorid, 3,5-Diamino-4-hydroxybrenzcatechin-sulfat, aromatische Nitrile, Nitrogruppen-haltige Aminoverbindungen, wie 3-Amino-6-methylamino-2-nitro-pyridin, Pikraminsäure, 1-(2(3)-Nitro-4-amino)-phenylazo-2-hydroxy-7-trimethytammoniumnaphthalin-chlorid, 1-Hydroxy-2-amino-4,6-dinitro-benzol, 1-Amino-2-nitro-4-bis-(2-hydroxyethyl)-aminobenzol, 1-Amino-2-(2-hydroxyethyl)-amino-5-nitro-benzol (HC Yellow Nr. 5), 1-Amino-2-nitro-4-(2-hydroxyethyt)-aminobenzo) (HC Red Nr. 7), 2-Chloro-5-nitro-N-hydroxyethyl-1,4-phenylendiamin, 1-(2-Hydroxyethyl)-amino-2-nitro-4-amino-benzol (HC Red Nr. 3), 4-Amino-3-nitrophenol, 4-Amino-2-nitrophenol, 6-Nitro-o-toluldin, 1-Amino-3-methyl-4-(2-hydroxyethyl)-amino-6-nitrobenzol (HC Violet Nr. 1), 1-Amino-2-nitro-4-(2,3-dihydroxypropyl)-amino-5-chlorobenzol (HC Red Nr. 10), 4-Amino-2-nitrodiphenyl-amino-2'-carbonsäure (2-(4-Amino-2-nitroanilino)-benzoesäure), 6-Nitro-2,5-d)aminopyridin, 2-Amino-6-chloro-4-nitrophenol, 2-Amino-6-chloro-4-nitrophenol, 1-Amino-2-(3-nitrophenylazo)-7-phenylazo-8-naphthol-3,6-disulfonsäure, Dinatriumsalz (Acid blue Nr. 29), 1-Amino-2-(2-hydroxy-4-nitrophenylazo)-8-naphthol-3,6-disulfonsäure, Dinatriumsalz (Palatincisrome green), 1-Amino-2-(3-chlor-2-hydroxy-5-nitrophenylazo)-8-naphthol-3,6-disuifonsäure, Dinatriumsalz (Gallion), 4-Amino-4'-nitrostilben-2,2'-disulfonsäure, Dinatriumsalz, 2,4-Diamino-3',5'-dinitro-2'hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)-ethylamino]-5-nitroanilin, 3-Amino-4-hydroxy-5-nitrobenzolsulfonsäure, (Hydrat), 3-Amino-3'-nitrobiphenyf, 3-Amino-4-nitro-acenaphthen, 2-Amino-1-nitronaphthalin, 5-Amino-6-nitrobenzo-1,3-dioxol, 1,4-Bis-(4-aminophenyl)-1,4-diazacycloheptan, Bis-(5-amino-2-hydroxyphenyl)-methan,Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest wie 4,4'-Diaminostilbendihydrochlorid, 4,4'-Diaminostilben-2,2'-disulfonsäure, Na-Salz, 4,4-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy).;propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin-trihydrochlorid, stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, -3-Amino-, 3-Amino-5-hydroxypyrazol, 4,5-Diamino-1-(2-hydroxyethyl)-pyrazol, 2-,3-, 8-Aminochinolin, 4-Amino-chinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin, 4-Hydroxyindolin und die in DE-U1-299 08 573 offenbarten Hydroxypyrimidine, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure,
Aminosäuren ausgewählt aus der Gruppe Arginin, Histidin, Tyrosin, Phenylalanin, β-Alanin, DOPA (Dihydroxyphenylalanin), Omithin, Lysin, Prolin und Tryptophan, Oligopeptide ausgewählt aus Glutathion oder die in den Hydrolysaten von Kollagen, Keratin, Casein, Elastin, Sojaprotein, Weizengluten oder Mandelprotein enthaltene Oligopeptide,
aromatischen Aldehyden ausgewählt aus der Gruppe Salicylaldehyd, 3-Hydroxybenzaldehyd, 4-Hydroxybenzaldehyd, o-Anisaldehyd, m-Anisaldehyd, p-Anisaldehyd, 4-Hydroxy-3-methylbenzaldehyd, 2-Hydroxy-3-methylbenzaldehyd, 2-Hydroxy-5-methylbenzaldehyd, 2-Hydroxy-4-methylbenzaldehyd, 2,3-Dihydroxybenzaldehyd, 2,5-Dihydroxybenzaldehyd, 3,4-Dihydroxybenzaldehyd, 2,4-Dihydroxybenzaldehyd, 3,5- Dihydroxybenzaldehyd, 2,3-Dihydrobenzo[b]furan 5-carboxaldehyd, Piperonal, 4-Ethoxy-benzaldehyd, 3,5-Dimethyl-4-hydroxybenzaldehyd, Vanillin, Isovanillin, 2,3,4-Trihydroxybenzaldehyd, 2,4,5- Trihydroxybenzaldehyd, 2,4,6-Trihydroxybenzaldehyd, 3,4,5-Trihydroxybenzaldehyd, 3-Chlor-4-hydroxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,4-Dihydroxy-5-methoxybenzaldehyd, 2-Hydroxy-1-naphthaldehyd, 4-Hydroxy-1-naphthaldehyd, 1-Hydroxy-2-naphthaldehyd, 2-Methoxy-1-naphthaldehyd, 4-Methoxy-1-naphthaldehyd, 2-Hydroxyacetophenon, 4-Hydroxyacetophenon, 2,4-Dihydroxyacetophenon, 2-Hydroxyzimtaldehyd, 4-Hydroxyzimtaldehyd, 2,4-Dihydroxyzimtaldehyd, 4-Hydroxy-benzylidenaceton, 4-Hydroxy-3-methoxybenzylidenaceton, 4-Hydroxy-3-methoxyzimtaldehyd (Coniferylaldehyd), 3,5-Dimethoxy-4-hydroxyzimtaldehyd, 4-Hydroxycinnamylidenacetaldehyd, 4-Methoxycinnamylidenacetaldehyd.

6. Mittel nach Anspruch 5, **dadurch gekennzeichnet, dass** die weitere Verbindung ausgewählt ist aus der Gruppe bestehend aus N-(2-Hydroxyethyl)-N-ethyl-, 2-Chlor-p-phenylendiamin, N,N-Bis-(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Arninomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6 triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin, β-Alanin, L-Prolin, L-Lysin, DL-Tyrosin sowie jeweils aus den vorzugsweise mit anorganischen Säuren gebildeten physiologisch verträglichen Salzen dieser Verbindungen.

7. Mittel nach einem der Ansprüche 1 bis 6, **dadurch gekennzeichnet, dass** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

8. Mittel nach einem der Ansprüche 1 bis 7, **dadurch gekennzeichnet, dass** es direkt ziehende Farbstoffe aus der Gruppe der Nitrophenylendiamine, Nitroaminophenole, Anthrachinone oder indophenole vorzugsweise in einer Menge von 0,01 bis 20 Gew.-%, bezogen auf das gesamte Färbemittel, enthält.

9. Mittel nach einem der Ansprüche 1 bis 8, **dadurch gekennzeichnet, dass** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

10. Mittel nach einem der Ansprüche 1 bis 9, **dadurch gekennzeichnet, dass** es Oxidationsmittel, insbesondere H₂O₂, in einer Menge von 0,01 bis 6 Gew.-%, bezogen auf die Anwendungslösung, enthält.

11. Mittel nach einem der Ansprüche 1 bis 10, **dadurch gekennzeichnet, dass** es anionische, zwitterionische oder nichtionische Tenside enthält.

12. Verwendung von Cyclohexenonderivaten mit der Formel I, 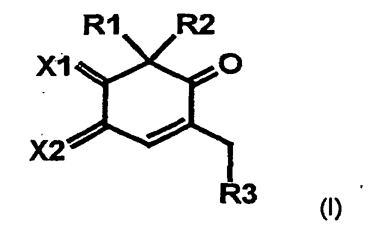 in der bedeuten
R¹, R², R³ unabhängig voneinander ein Wasserstoffatom oder eine C₁-C₄-Alkylgruppe, wobei R¹ und R² zusammen auch einen Ring bilden können, X¹ und X² ein Sauerstoff-, ein Schwefelatom oder gemeinsam eine 1,2-Arylendiiminogruppe, die ihrerseits im Aromaten durch Halogenatome, C₁-C₄-Alkyl-, C₁-C₄ Alkoxy-, Nitro-, Amino-, C₁-C₄-Alkylamino-, Hydroxy-, Carboxy-, Sulfogruppen oder einen weiteren kondensierten aromatischen Ring substituiert und an einem der Stickstoffatome durch eine C₁-C₆-Alkyl-, Aralkyl-, Aryl-, C₂-C₄-Alkenyl-, C₁-C₈-Hydroxyalkyl- oder Carboxyalkylgruppe quaterniert sein kann,
wobei der weitere kondensierte aromatische Ring durch Halogenatome, in Halogenatome, C₁-C₄-Alkyl-, C₁-C₄-Alkoxy-, Nitro-, Amino-, C₁-C₄-Alkylamino-, Hydroxy-, Carboxy-, Sulfogruppen substituiert sein kann,
oder dessen tautomeren Formen oder deren physiologisch verträglichen Salzen als eine färbende Komponente in Oxidationshaarfärbemitteln.

13. Verfahren zum Färben von keratinhaltigen Fasern, insbesondere menschlichen Haaren, worin ein Färbemittel, enthaltend
A) mindestens ein Cyclohexenonderivat mit der Formel I 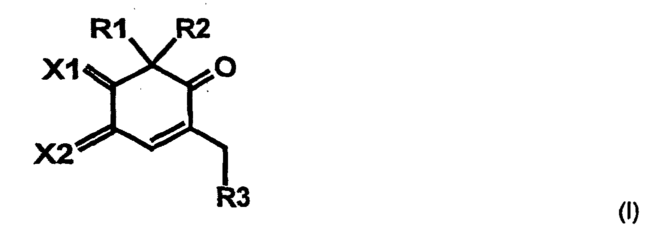 in der bedeuten
R¹, R², R³ unabhängig voneinander ein Wasserstoffatom oder eine C₁-C₄-Alkylgruppe, wobei R¹ und R2 zusammen auch einen Ring bilden können,
X¹ und X² ein Sauerstoff-, ein Schwefelatom oder gemeinsam eine 1,2-Arylendiiminogruppe, die ihrerseits im Aromaten durch Halogenatome, C₁-C₄-Alkyl-, C₁-C₄-Alkoxy-, Nitro-, Amino-, C₁-C₄-Alkylamino-, Hydroxy-, Carboxy-, Sulfogruppen oder einen weiteren kondensierten aromatischen Ring substituiert und an einem der Stickstoffatome durch eine C₁-C₆-Alkyl-, Aralkyl-, Aryl-, C₂-C₄ Alkenyl- oder C₁-C₆-Hydroxyalkyl- Carboxyalkylgruppe, quaterniert sein kann,
wobei der weitere kondensierte aromatische Ring durch Halogenatome, C₁-C₄-Alkyl-, C₁-C₄-Alkoxy-, Nitro-, Amino-, C₁-C₄-Alkylamino-, Hydroxy-, Carboxy-, Sulfogruppen substituiert sein kann
oder dessen tautomere Formen oder dessen physiologisch verträgliche Salze,
B mindestens eine Verbindung ausgewählt aus Verbindungen mit primärer oder sekundärer Aminogruppe oder Hydroxygruppe, ausgewählt aus primären oder sekundären aromatischen Aminen, stickstoffhattigen heterocyclischen Verbindungen und aromatischen Hydroxyverbindungen, Aminosäuren, aus 2 bis 9 Aminosäuren aufgebauten Oligopeptiden und/oder aromatischen Aldehyden;
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Preparation for colouring keratin-containing fibres, more particularly human hair, containing as a colouring component at least one cyclohexenone derivative corresponding to formula (I): 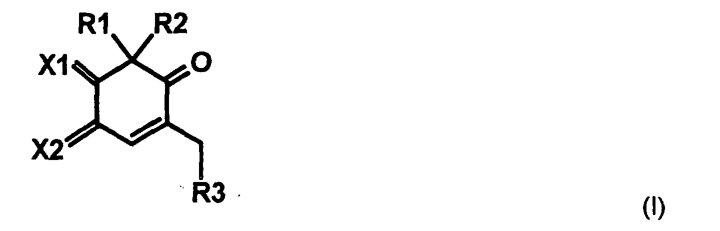 in which
R¹, R² and R³ independently of one another represent a hydrogen atom or a C₁₋₄ alkyl group and R¹ and R² together may also form a ring,
X¹ and X² represent an oxygen atom, a sulfur atom or, together, a 1,2-arylenediimino group which, in turn, may be substituted in the aromatic ring by halogen atoms, C₁₋₄ alkyl, C₁₋₄ alkoxy, nitro, amino, C₁₋₄ alkylamino, hydroxy, carboxy, sulfo groups or another condensed aromatic ring and quaternized at one of the nitrogen atoms by a C₁₋₆ alkyl, aralkyl, aryl, C₂₋₄ alkenyl, C₁₋₆ hydroxyalkyl or carboxyalkyl group;
the other condensed aromatic ring may be substituted by halogen atoms, C₁₋₄ alkyl, C₁₋₄ alkoxy, nitro, amino, C₁₋₄ alkylamino, hydroxy, carboxy or sulfo groups,
or tautomeric forms thereof or physiologically compatible salts thereof.

2. Preparation as claimed in claim 1, **characterized in that** 3,3,5-trimethylcyclohex-5-ene-1,2,4-trione, 3,5-dibutyl-2-hydroxy-2,5-cyclohexadiene-1,4-dione, 2-ethyl-5-hydroxy-2,5-cyclohexadiene-1,4-dione, 2-hydroxy-3-methylphenazine, 3,10-dimethyl-2(10H)-phenazinone, 3-methyl-2,8-phenazinediol, 3,7-dihydroxy-2,5-dimethylphenazinium methosulfate, 1,7-dimethyl-2,8-phenazinediol, 3,7-dihydroxy-2,8-dimethyl-5-phenylphenazinium sulfate, 1,1,3,5-tetramethyl-2-oxo-1,2-dihydrophenazinium tetrafluoroborate, 1,1,3,5-tetramethyl-7,8-dimethoxy-1,2-dihydro-2-oxophenazninium tetrafluoroborate, 1,1,3,5-tetramethyl-2-oxo-8-nitro-1,2-dihydrophenazinium tetrafluorborate, 1,1,3-trimethyl-2-oxo-8-nitro-1,2-dihydrophenazinium tetrafuroborate, 1,1,3-trimethyl-5-phenyl-1,2-dihydro-2-oxophenazinium tetrafluoroborate, 8-chloro-1,1,3,5-tetramethyl-2-oxo-1,2-dihydrophenazinium tetrafluoroborate, 8-chloro-5-isopropyl-1,1,3-trimethyl-2-oxo-1,2-dihydrophenazinium tetrafluorborate, p-toluenesulfonate, iodide, bromide, chloride, sulfate, methylsulfate, tetrachlorozincate, perchlorate, perfluoromethanesulfonate, methanesulfonate, benzenesulfonate and mixtures thereof are used as the compounds of formula (I).

3. Preparation as claimed in claim 1 or 2, **characterized in that** the cyclohexenone derivatives of formula (I) are used in a quantity of 0.03 to 65 mmol and more particularly 1 to 40 mmol, based on 100 g of the colorant as a whole.

4. Preparation as claimed in any of claims 1 to 3, **characterized in that** it additionally contains at at least one compound selected from compounds containing a primary or secondary amino group or hydroxy group selected from primary or secondary aromatic amines, nitrogen-containing heterocyclic compounds and aromatic hydroxy compounds, amino acids, oligopeptides of 2 to 9 amino acids and/or aromatic aldehydes.

5. Preparation as claimed in claim 4, **characterized in that** the other compound is selected from primary or secondary amines from the group consisting of N-(2-hdroxyethyl)-N-ethyl-, N-(2-methoxyethyl-), 2,3-, 2,4-, 2,5-dichloro-p-phenylenediamine, 2-chloro-p-phenylenediamine, N,N-bis-(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)-ethanol, 2,5-diaminotoluene, phenol, phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 5-(2-hydroxyethylamino)-4-methoxy-2-methyl-, 4-amino-2-aminomethyl phenol, 2-hydroxymethyl-4-aminophenol, 2-diethylaminomethyl-4-aminophenol, 2-dimethylaminomethyl-4-aminophenol, 2,6-dichloro-4-aminophenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulfonic acid, 3-amino-4-hydroxybenzenesulfonic acid, 4-amino-3-hydroxynaphthalene sulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulfate, 4,6-diaminopyrogallol dihydrochloride, 3,5-diamino-4-hydroxypyrocatechol sulfate, aromatic nitriles, amino compounds containing nitro groups, such as 3-amino-6-methylamino-2-nitropyridine, picramic acid, 1-(2(3)-nitro-4-amino)-phenylazo-2-hydroxy-7-trimethylammonium naphthalene chloride, 1-hydroxy-2-amino-4,6-dinitro-benzene, 1-amino-2-nitro-4-bis-(2-hydroxyethyl)-aminobenzene, 1-amino-2-(2-hydroxyethyl)-amino-5-nitrobenzene (HC Yellow No. 5), 1-amino-2-nitro-4-(2-hydroxyethyl)-aminobenzene (HC Red No. 7), 2-chloro-5-nitro-N-hydroxyethyl-1,4-phenylenediamine, 1-(2-hydroxyethyl)-amino-2-nitro-4-aminobenzene (HC Red No. 3), 4-amino-3-nitrophenol, 4-amino-2-nitrophenol, 6-nitro-o-toluidine, -1-amino-3-methyl-4-(2-hydroxyethyl)-amino-6-nitrobenzene (HC Violet No. 1), 1-amino-2-nitro-4-(2,3-dihydroxypropyl)-amino-5-chlorobenzene (HC Red No. 10), 4-amino-2-nitrodiphenylamino-2'-carboxylic acid (2-(4-amino-2-nitroanilino)-benzoic acid), 6-nitro-2,5-diaminopyridine, 2-amino-6-chloro-4-nitrophenol, 2-amino-6-chloro-4-nitrophenol, 1-amino-2-(3-nitrophenylazo)-7-phenylazo-8-naphthol-3,6-disulfonic acid, disodium salt (Acid blue No. 29), 1-amino-2-(2-hydroxy-4-nitrophenylazo)-8-naphthol-3,6-disulfonic acid, disodium salt (Palatinchrome green), 1-amino-2-(3-chlor-2-hydroxy-5-nitrophenylazo)-8-naphthol-3,6-disulfonic acid, disodium salt (Gallion), 4-amino-4'-nitrostilbene-2,2'-disulfonic acid, disodium salt, 2,4-diamino-3',5'-dinitro-2'-hydroxy-5-methylazobenzene (Mordant brown 4), 4'-amino-4-nitrodiphenylamine-2-sulfonic acid, 4'-amino-3'-nitrobenzophenone-2-carboxylic acid, 1-amino-4-nitro-2-(2-nitrobenzylideneamino)-benzene, 2-[2-(diethylamino)-ethylamino]-5-nitroaniline, 3-amino-4-hydroxy-5-nitrobenzenesulfonic acid, (hydrate), 3-amino-3'-nitrobiphenyl, 3-amino-4-nitroacenaphthene, 2-amino-1-nitronaphthalene, 5-amino-6-nitrobenzo-1,3-dioxol, 1,4-bis-(4-aminophenyl)-1,4-diazacycloheptane, bis-(5-amino-2-hydroxyphenyl)-methane, aniline, more particularly anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)-benzene, 2-nitro-1-amino-4-[bis-(2-hydroxyethyl)-amino]-benzene, 4-amino-2-nitrodiphenylamine-2'-carboxylic acid, 2-amino-6-chloro-4-nitrophenol, 1-amino-5-chloro-4-(2-hydroxyethylamino)-2-nitrobenzene, anilines, more particularly anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)-benzene, 2-nitro-1-amino-4-[bis-(2-hydroxyethyl)-amino]-benzene, 4-amino-2-nitrodiphenylamine-2'-carboxylic acid, 2-amino-6-chloro-4-nitrophenol, 1-amino-5-chloro-4-(2-hydroxyethylamino)-2-nitrobenzene, aromatic anilines and phenols containing another aromatic group, such as 4,4'-diaminostilbene dihydrochloride, 4,4'-diaminostilbene-2,2'-disulfonic acid, Na salt, 4,4'-diaminodiphenylmethane, sulfide, sulfoxide, amine, 4,4'-diaminodiphenylamine-2-sulfonic acid, 4,4'-diaminobenzophenone, diphenylether, 3,3',4,4'-tetraaminodiphenyl tetrahydrochloride, 3,3',4,4'-tetraaminobenzophenone, 1,3-bis-(2,4-diaminophenoxy)-propane tetrahydrochloride, 1,8-bis-(2,5-diaminophenoxy)-3,6-dioxaoctane tetrahydrochloride, 1,3-bis-(4-aminophenylamino)-propane, 2-propanol, 1,3-bis-[N-(4-aminophenyl)-2-hydroxyethylamino]-2-propanol, N,N-bis-[2-(4-aminophenoxy)-ethyl]methylamine trihydrochloride,
nitrogen-containing heterocyclic compounds selected from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methyl -pyrimidine, 2,3,4-trimethylpyrrole, 2,4-dimethyl-3-ethylpyrroel, 3,5-diaminopyrazole, -1,2,4-triazole, 3-amino-, 3-amino-5-hydroxypyrazole, 4,5-diamino-1-(2-hydroxyethyl)-pyrazole, 2-,3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline and indole and indoline derivatives, such as 4-, 5-, 6-, 7-aminoindole, 4-, 5-, 6-, 7-hydroxyindole, 5,6-dihydroxyindole, 5,6-dihydroxyindoline, 4-hydroxyindoline and the hydroxypyrimidines disclosed in DE-U1-299 08 573 and physiologically compatible salts of these compounds formed with preferably inorganic acids,
aromatic hydroxy compounds selected from the group consisting of 2-, 4-, 5-methylresorcinol, 2,5-dimethylresorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulfonic acid, 3,6-dihydroxy-2,7-naphthalenesulfonic acid,
amino acids selected from the group consisting of arginine, histidine, tyrosine, phenylalanine, β-alanine, DOPA (dihydroxyphenylalanine), ornithine, lysine, proline and tryptophane,
oligopeptides selected from glutathione or the oligopeptides present in the hydrolyzates of collagen, keratin, casein, elastin, soya protein, wheat gluten or almond protein,
aromatic aldehydes selected from the group consisting of salicylaldehyde, 3-hydroxybenzaldehyde, 4-hydroxybenzaldehyde, o-anisaldehyde, m-anisaldehyde, p-anisaldehyde, 4-hydroxy-3-methylbenzaldehyde, 2-hydroxy-3-methylbenzaldehyde, ' 2-hydroxy-5-methylbenzaldehyde, 2-hydroxy-4-methylbenzaldehyde, 2,3-dihydroxybenzaldehyde, 2,5-dihydroxybenzaldehyde, 3,4-dihydroxybenzaldehyde, 2,4-dihydroxybenzaldehyde, 3,5-dihydroxybenzaldehyde, 2,3-dihydrobenzo[b]furan-5-carboxaldehyde, piperonal, 4-ethoxybenzaldehyde, 3,5-dimethyl-4-hydroxybenzaldehyde, vanillin, isovanillin, 2,3,4-trihydroxybenzaldehyde, 2,4,5-trihydroxybenzaldehyde, 2,4,6-trihydroxybenzaldehyde, 3,4,5-trihydroxybenzaldehyde, 3-chloro-4-hydroxybenzaldehyde, 2,4-dimethoxybenzaldehyde, 2,5-dimethoxybenzaldehyde, 2,6-dimethoxybenzaldehyde, 3,4-dimethoxybenzaldehyde, 3,4-dihydroxy-5-methoxybenzaldehyde, 2-hydroxy-1-naphthaldehyde, 4-hydroxy-1 -naphthaldehyde, 1-hydroxy-2-naphthaldehyde, 2-methoxy-1-naphthaldehyde, 4-methoxy-1-naphthaldehyde, 2-hydroxyacetophenone, 4-hydroxyacetophenone, 2,4-dihydroxyacetophenone, 2-hydroxycinnamaldehyde, 4-hydroxycinnamaldehyde, 2,4-dihydroxycinnamaldehyde, 4-hydroxybenzylidene acetone, 4-hydroxy-3-methoxybenzylidene acetone, 4-hydroxy-3-methoxycinnamaldehyde (coniferyl aldehyde), 3,5-dimethoxy-4-hydroxycinnamaldehyde, 4-hydroxycinnamylidene acetaldehyde, 4-methoxycinnamylidene acetaldehyde.

6. Preparation as claimed in claim 5, **characterized in that** the other compound is selected from the group consisting of N-(2-hydroxyethyl)-N-ethyl-, 2-chloro-p-phenylenediamine, N,N-bis-(2-hydroxyethyl)-p-phenylenediamine, 4-aminophenol, p-phenylenediamine, 2-(2,5-diaminophenyl)-ethanol, 2,5-diaminotoluene, 3,4-methylenedioxyaniline, 2-amino-4-(2-hydroxyethylamino)-anisole, 2-(2,4-diaminophenoxy)-ethanol, 3-amino-2,4-dichloro-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 2-aminomethyl-4-aminophenoi, 2-diethylaminomethyl-4-aminophenol, 2-dimethylaminomethyl-4-aminophenol, 2,6-dichloro-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridin, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 5,6-dihydroxyindole and 5,6-dihydroxyindoline, β-alanine, L-proline, L-lysine, DL-tyrosine and the physiclogically compatible salts of these compounds formed preferably with inorganic acids.

7. Preparation as claimed in any of claims 1 to 6, **characterized in that** it contains colour intensifiers selected from the group consisting of piperidine, piperidine-2-carboxylic acid, piperidine-3-carboxylic acid, piperidine-4-carboxylic acid, pyridine, 2-hydroxypyridined, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or mixtures thereof.

8. Preparation as claimed in any of claims 1 to 7, **characterized in that** it contains substantive dyes from the group of nitrophenylenediamines, nitroaminopenols, anthraquinones or indophenols, preferably in a quantity of 0.01 to 20% by weight, based on the colorant as a whole.

9. Preparation as claimed in any of claims 1 to 8, **characterized in that** ammonium or metal salts selected from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc are added.

10. Preparation as claimed in any of claims 1 to 9, **characterized in that** it contains oxidizing agents, more particularly H₂O₂, in a quantity of 0.01 to 6% by weight, based on the solution applied.

11. Preparation as claimed in any of claims 1 to 8, **characterized in that** it contains anionic, zwitterionic or nonionic surfactants.

12. The use of cyclohexene derivatives corresponding to formula (I): 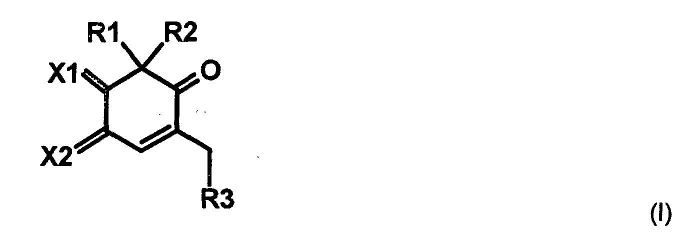 in which
R¹, R² and R³ independently of one another represent a hydrogen atom or a C₁₋₄ alkyl group and R¹ and R² together may also form a ring,
X¹ and X² represent an oxygen atom, a sulfur atom or, together, a 1,2-arylenediimino group which, in turn, may be substituted in the aromatic ring by halogen atoms, C₁₋₄ alkyl, C₁₋₄ alkoxy, nitro, amino, C₁₋₄ alkylamino, hydroxy, carboxy, sulfo groups or another condensed aromatic ring and quaternized at one of the nitrogen atoms by a C₁₋₆ alkyl, aralkyl, aryl, C₂₋₄ alkenyl, C₁₋₆ hydroxyalkyl or carboxyalkyl group;
the other condensed aromatic ring may be substituted by halogen atoms, C₁₋₄ alkyl, C₁₋₄ alkoxy, nitro, amino, C₁₋₄ alkylamino, hydroxy, carboxy or sulfo groups,
or tautomeric forms thereof or physiologically compatible salts thereof as a colouring component in oxidation hair colorants.

13. A process for colouring keratin fibres, more particularly human hair, in which a colorant containing
A) at least one cyclohexenone derivative corresponding to formula (I): 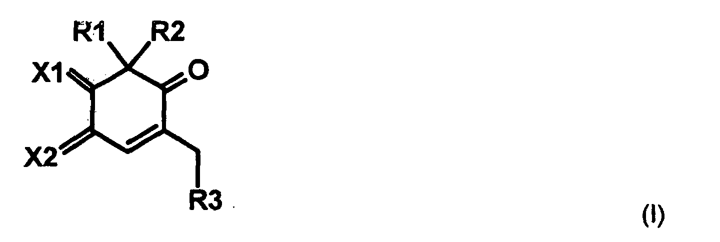 in which
R¹, R² and R³ independently of one another represent a hydrogen atom or a C₁₋₄ alkyl group and R¹ and R² together may also form a ring,
X¹ and X² represent an oxygen atom, a sulfur atom or, together, a 1,2-arylenediimino group which, in turn, may be substituted in the aromatic ring by halogen atoms, C₁₋₄ alkyl, C₁₋₄ alkoxy, nitro, amino, C₁₋₄ alkylamino, hydroxy, carboxy, sulfo groups or another condensed aromatic ring and quaternized at one of the nitrogen atoms by a C₁₋₆ alkyl, aralkyl, aryl, C₂₋₄ alkenyl, C₁₋₆ hydroxyalkyl or carboxyalkyl group; the other condensed aromatic ring may be substituted by halogen atoms, C₁₋₄ alkyl, C₁₋₄ alkoxy, nitro, amino, C₁₋₄ alkylamino, hydroxy, carboxy or sulfo groups,
or tautomeric forms thereof or physiologically compatible salts thereof,
B) at least one compound selected from compounds containing a primary or secondary amino group or hydroxy group selected from primary or secondary aromatic amines, nitrogen-containing heterocyclic compounds and aromatic hydroxy compounds, amino acids, oligopeptides of 2 to 9 amino acids and/or aromatic aldehydes
and typical cosmetic ingredients is applied to the keratin-containing fibres, left thereon for a while, usually about 30 minutes, and then rinsed out or washed out with a shampoo.

## Revendications

1. Agent pour teindre des fibres kératiniques, en particulier les cheveux humains, contenant en tant que composant teignant, au moins un dérivé cyclohexénone de formule I, 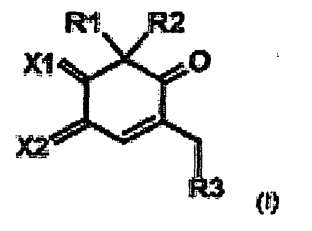 dans laquelle
R¹, R², R³ représentent indépendamment l'un de l'autre, un atome d'hydrogène, ou un groupe alkyle en C₁ à C₄, R¹ et R² pouvant former également ensemble un cycle,
X¹ et X² représentent un atome d'oxygène, un atome de soufre ou conjointement un groupe 1,2-arylène-diimino, qui, de son côté peut être substitué dans le groupe aromatique par des atomes d'halogène, des groupes alkyle en C₁ à C₄, alcoxy en C₁ à C₄, nitro, amino, alkylamino en C₁ à C₄, hydroxy, carboxy, sulfo, ou un noyau aromatique condensé supplémentaire, et peut être quaternisé sur un des atomes d'azote par un groupe alkyle C₁ à C₆, aralkyle, aryle, alcényle en C₂ à C₄, hydroxyalkyle en C₁ à C₆ ou carboxyalkyle
le noyau aromatique condensé supplémentaire pouvant être substitué par des atomes d'halogène, des groupes alkyle en C₁ à C₄, alcoxy en C₁ à C₄, nitro, amino, alkylamino en C₁ à C₄, hydroxy, carboxy, sulfo,
ou ses formes tautomères ou ses sels acceptables sur le plan physiologique.

2. Agent selon la revendication 1, **caractérisé en ce que**, en tant que composés de formule I, la 3,3,5-triméthylcjrclohex-5-ène-1,2,4-trione, la 3,5-dibutyl-2-hydroxy-2,5-cyclohexadièn-1,4-dione, la 2-éthyl-5-hydroxy-2,5-cyclohexadiène-1,4-dione, la 2-hydroxy-3-méthylphénazine, la 3,10-diméthyl-2(10H)-phénazinone, le 3-méthyl-2,8-phénazinediol, le méthosulfate de 3,7-dihydroxy-2,5-diméthyl-phénazinium, le 1,7-diméthyl-2,8-phénazinediol, les sulfates de 3,7-dihydroxy-2,8-diméthyl-5-phénylphénazinium, le tétrafluoroborate de 1,1,3,5-tétraméthyl-2-oxo-1,2-dihydrophénazinium, de 1,1,3,5-tétraméthyl-7,8-diméthoxy-1,2-dihydro-2-oxophénazinium, le tétrafluoroborate de 1,1,3,5-tétraméthyl-2-oxo-8-nitro-1,2-dihydrophénazinium, le tétrafluoroborate de 1,1,3-triméthyl-2-oxo-8-nitro-1,2-dihydrophénazinium, de 1,1,3-triméthyl-5-phényl-1,2-dihydro-2-oxophénazinium, le tétrafluoroborate de 8-chloro-1,1,3,5-tétraméthyl-2-oxo-1,2-dihydrophénazinium, de 8-chloro-5-isopropyl-1,1,3-triméthyl-2-oxo-1,2-dihydrophénazinium, le p-toluènesuffonate, iodure, bromure, chlorure, sulfate, méthylsulfate, tétrachlorozincate, perchlorate, perfluorométhanesulfonate, méthanesulfonate, benzènesulfonate, de 8-chloro-1,1,3,5-tétraméthyl-2-oxo-1,2-dihydrophénazinium, de 8-chloro-5-isopropyl-1,1,3-triméthyl-2-oxo-1,2-dihydrophénazinium, ainsi que leurs mélanges quelconques, sont mis en oeuvre.

3. Agent selon l'une quelconque des revendications 1 à 2, **caractérisé en ce que** les dérivés cyclohexénone de formule I sont contenus en une quantité de 0,03 à 65 mmoles, en particulier de 1 à 40 mmoles, par rapport aux 100 g de la teinture totale.

4. Agent selon l'une quelconque des revendications 1 à 3, **caractérisé en ce qu'**il contient en outre au moins un composé, choisi parmi les composés ayant un groupe amino primaire ou secondaire, ou un groupe hydroxy, choisi parmi les amines aromatiques primaires ou secondaires, les composés hétérocycliques azotés, et les composés hydroxyaromatiques, les acides aminés, les oligopeptides construits à partir de 2 à 9 acides aminés, et/ou les aldéhydes aromatiques.

5. Agent selon la revendication 4, **caractérisé en ce que** le composé supplémentaire est choisi parmi
les amines primaires ou secondaires issues du groupe formé par la N-(2-hydroxyéthyl)-N-éthyl-, N-(2-méthoxyéthyl-), 2,3-, 2,4-, 2,5-dichloro-p-phénylènediamine, la 2-chloro-p-phénylènediamine, la N,N-bis-(2-hydroxyéthyl)-p-phénylènediamine, le dibromhydrate de 2,5-dihydroxy-4-morpholinoaniline, le 2-, 3-, 4-aminophénol, la o-, m-, p-phénylènediamine, le 2,4-diaminophénoxyéthanol, le 2-(2,5-diaminophényl)-éthanol, le 2,5-diaminotoluène, -phénol, -phénétol, la 4-méthylamino-, 3-amino-4-(2'-hydroxyéthyloxy)-, 3,4-méthylènediamino-, 3,4-méthylènedioxyaniline, le 3-amino-2,4-dichloro-, 4-méthylamino-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 5-(2-hydroxyéthylamino)-4-méthoxy-2-méthyl-, 4-amino-2-aminométhyl-phénol, le 2-hydroxyméthyl-4-aminophénol, le 2-diéthylaminométhyl-4-aminophénol, le 2-diméthylamhométhyl-4-aminophénol, le 2,6-dichloro-4-aminophénol, le 1,3-diamino-2,4-diméthoxybenzène, l'acide 2-, 3-, 4-aminobenzoïque, -phénylacétique, l'acide 2,3-, 2,4-, 2,5-, 3,4-, 3,5-diaminobenzoïque, l'acide 4-, 5-aminosalicylique, l'acide 3-amino-4-hydroxy-, 4-amino-3-hydroxy-benzoïque, l'acide 2-, 3-, 4-aminobenzènesulfonique, l'acide 3-amino-4-hydroxybenzènesulfonique, l'acide 4-amino-3-hydroxynaphtalènesulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-napthoïque, l'acide 3-aminophtalique, l'acide 5-aminoisophtalique, le 1,3,5-, 1,2,4-triaminobenzène, le tétrachlorhydrate de 1,2,4,5-tétraaminobenzène, le trichlorhydrate de 2,4,5-triaminophénol, le pentachlorhydrate de pentaaminobenzène, l'hexachlorhydrate d'hexaaminobenzène, le trichlorhydrate de 2,4,6-triaminorésorcine, le sulfate de 4,5-diaminopyrocatéchol, le dichlorhydrate de 4,6-diaminopyrogallol, le sulfate de 3,5-diamino-4-hydroxypyrocatéchol, les nitriles aromatiques, les composés amino contenant des groupes nitro, comme la 3-amino-6-méthylamino-2-nitropyridine, l'acide picramique, le chlorure de 1-(2(3)-nitro-4-amino)-phénylazo-2-hydroxy-7-triméthylammonium-naphtalène, le 1-hydroxy-2-amino-4,6-dinitrobenzène, le 1-amino-2-nitro-4-bis-(2-hydroxyéthyl)-amino-benzène, le 1-amino-2-(2-hydroxyéthyl)-amino-5-nitrobenzène (HC Yellow N° 5), le 1-amino-2-nitro-4-(2-hydroxyéthyl)-aminobenzène (HC Red N° 7) la 2-chloro-5-nitro-N-hydroxyéthyl-1,4-phénylènediamine, le 1-(2-hydroxyéthyl)-amino-2-nitro-4-aminobenzène (HC Red N° 3) , le 4-amino-3-nitrophénol, le 4-amino-2-nitrophénol, la 6-nitro-o-toluidine, le 1-amino-3-méyhyl-4-(2-hydroxyéthyl)-amino-6-nitrobenzène (HC Violet N° 1), le 1-amino-2-nitro-4-(2,3-dihydroxypropyl)-amino-5-chlorobenzène (HC Red N° 10), l'acide 4-amino-2-nitrodiphényl-amino-2'-carboxylique (l'acide 2-(4-amino-2-nitroanilino)-benzoïque), la 6-nitro-2,5-diaminopyridine, le 2-amino-6-chloro-4-nitrophénol, le 2-amino-6-chloro-4-nitrophénol, l'acide 1-amino-2-(3-nitrophénylazo) 7-phénylazo-8-naphtol-3,6-disulfonique, sel disodique (Acid Blue N° 29), l'acide 1-amino-2-(2-hydroxy-4-nitrophénylazo)-8-naphtol-3,6-disulfonique, sel disodique (Palatinchrome Green), l'acide 1-amino-2-(3-chloro-2-hydroxy-5-nitrophénylazo)-8-naphtol-3,6-disulfonique, sel disodique (Gallion), l'acide 4-amino-4-nitrostilbène-2,2'-disulfonique, sel disodique, le 2,4-diamino-3',5'-dinitro-2'-hydroxy-5-méthyl-azobenzène (Mordant Brown 4), l'acide 4'-amino-4-nitrodiphénylamine-2-sulfonique, l'acide 4'-amino-3'-nitrobenzophénone-2-carboxylique, le 1-amino-4-nitro-2-(2-nitrobenzylidèneamino)-benzène, la 2-[2-(diéthylamino)-éthylamino]-5-nitroaniline, l'acide 3-amino-4-hydroxy-5-nitrobenzènesulfonique, (hydrate), le 3-amino-3'-nitrobiphényle, le 3-amino-4-nitro-acénaphtène, le 2-amino-1-nitronaphtalène, le 5-amino-6-nitrobenzo-1,3-dioxol, le 1,4-bis-(4-aminophényl)-1,4-diazacycloheptane, le bis-(5-amino-2-hydroxyphényl)-méthane, les anilines, en particulier les anilines contenant des groupes nitro, comme la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthy)amino)-benzène, le 2-nitro-1-amino-4[bis-(2-hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2-hydroxyéthylamino)-2-nitrobenzène, les anilines en particulier les anilines, contenant des groupes nitro, comme la 4-nitroaniline, la 2-nitroaniline le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)-benzène, le 2-nitro-1-amino-4-[bis-(2 hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2' hydroxyéthylamino)-2-nitrobenzène, les anilines aromatiques ou les phénols avec un résidu aromatique supplémentaire tels que le dichlorhydrate de 4,4'-diaminostilbène, l'acide 4,4'-diaminostilbène-2,2'-disulfonique, sel sodique, le 4,-4'-diaminodiphényl-méthane, -sulfure, -sulfoxyde, -amine, l'acide 4,4'-diaminodiphénylamine-2-sulfonique, la 4,4'-diaminobenzophénone, l'éther diphénylique de celle-ci, le tétrachlorhydrate de 3,3',4,4'-tétraaminodiphényle, la 3,3',4,4'-tétraaminobenzophénone, le tétrachlorhydrate de 1,3-bis-(2,4-diaminophénoxy)-propane, le tétrachlorhydrate de 1,8-bis-(2,5-diamino-phénoxy)-3,6-dioxaoctane, le 1,3-bis-(4-aminophénylamino)-propane, -2-propanol, le 1,3-bis-[N-(4-aminophényl)-2-hydroxyéthylamino]-2-propanol, le trichlorhydrate de N,N-bis-[2-(4-aminophénoxy)-éthyl]méthylamine, les composés hétérocycliques azotés choisis dans le groupe formé par la 2-, 3-, 4-amino-,2-amino-3-hydroxy-, 2,6-diamino-, 2,5-diamino-, 2,3-diamino-, 2-diméthylamino-5-amino-, 3-amino-2-méthylamino-6-méthoxy-, 2,3-diamino-6-méthoxy-, 3,5-diamino-2,6-diméthoxy-, 2,4,5-triamino-, 2,6-dihydroxy-3,4-diméthylpyridine, la 4,5,6-triamino-, 2-hydroxy-4,5,6-triamino-, 4-hydroxy-2,5,6-triamino-, 2,4,5,6-tétraamino-, 2-méthylamino-4,5,6-triamino-, 2,4-, 4,5-diamino-, 2-amino-4-méthoxy-6-méthylpyrimidine, le 2,3,4-triméthylpyrrole, le 2,4-diméthyl-3-éthyl-pyrrole, le 3,5-diaminopyrazole, -1,2,4-triazole, le 3-amino-, 3-amino-5-hydroxypyrazole, le 4,5-diamino-1-(2-hydroxyéthyl)-pyrazole, la 2-,3-, 8-aminoquinoléine, la 4-amino-quinaldine, l'acide 2-, 6-amino-nicotinique, la 5-aminoisoquinoléine, le 5-, 6-aminoindazole, le 5-, 7-amino-benzimidazole, -benzothiazole, la 2,5-dihydroxy-4-morpholinoaniline, ainsi que les dérivés indole et indoline, comme le, 4-, 5-, 6-, 7-aminoindole, le 4-, 5-, 6-, 7-hydroxyindole, le 5,6-dihydroxyindole, la 5,6-dihydroxyindoline, la 4-hydroxyindoline, et les hydroxypyrimidines décrites dans le brevet DE-U1-299 08 573, ainsi qu'à chaque fois les sels de ces composés, acceptables sur le plan physiologique, formés avec des acides de préférence inorganiques,
les composés hydroxyaromatiques choisis dans le groupe, formé par la 2-, 4-, 5-méthylrésorcine, la 2,5-diméthylrésorcine, la résorcine, le 3-méthoxyphénol, le pyrocatéchol, l'hydroquinone, le pyrogallol, la phloroglucine, l'hydroxyhydroquinone, le 2-, 3-, 4-méthoxy-3-diméthylamino-, 2-(2-hydroxyéthyl)-, 3,4-méthylènedioxyphénol, l'acide 2,4-, 3,4-dihydroxybenzoïque, -phénylacétique, l'acide gallique, l'acide 2,4,6-trihydroxybenzoïque; -acétophénone, la 2-, 4-chlororésorcine, le 1-naphtol, le 1,5-, 2,3-, 2,7-dihydroxyhnaphtalène, l'acide 6-diméthylamino-4-hydroxy-2-naphtalènesulfonique, l'acide 3,6-dihydroxy-2-7-naphtalènesulfonique,
les aminoacides choisis dans le groupe formé par l'arginine, l'histidine, la tyrosine, la phénylalanine, la β-alanine, DOPA (la dihydroxyphénylalanine), l'ornithine, la lysine, la proline, et le tryptophane, les oligopeptides choisis parmi le glutathion ou les oligopeptides contenus dans les hydrolysats de collagène, de kératine, de caséine, d'élastine, de protéine de soja, de gluten du blé, ou de protéine d'amande, les aldéhydes aromatiques choisis dans le groupe formé par le salicylaldéhyde, le 3-hydroxybenzaldéhyde, le 4-hydroxybenzaldéhyde, l'o-anisaldéhyde, le m-anisaldéhyde, le p-anisaldéhyde, le 4-hydroxy-3-méthyibenzaldéhyde, le 2-hydroxy-3-méthylbenzaldéhyde, le 2-hydroxy-5-méthylbenzaldéhyde le 2-hydroxy-4-méthylbenzaldéhyde, le 2,3-dihydroxybenzaldéhyde, le 2,5-dihydroxybenzaldéhyde, le 3,4-dihydroxybenzaldéhyde, le 2,4-dihydroxybenzaldéhyde, le 3,5-dihydroxybenzaldéhyde, le 2,3-dihydrobenzo[b]furane-5-carboxaldéhyde, le pipéronal, le 4-éthoxy-benzaldéhyde, le 3,5-diméthyl-4-hydroxybenzaldéhyde, la vanilline, l'isovanilline, le 2,3,4-trihydroxybenzaldéhyde, le 2,4,5-trihydroxybenzaldéhyde, le 2,4,6-trihydroxybenzaldéhyde, le 3,4,5-trihydroxybenzaldéhyde, le 3-chloro-4-hydroxybenzaldéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,4-dihydroxy-5-méthoxybenzaldéhyde, le 2-hydroxy-1-naphtaldéhyde, le 4-hydroxy-1-naphtaldéhyde, le 1-hydroxy-2-naphtaldéhyde, le 2-méthoxy-1-naphtaldéhyde, le 4-méthoxy-1-naphtaldéhyde, la 2-hydroxyacétophénone, la 4-hydroxyacétophénone, la 2;4-dihydroxyacétophénone, le 2-hydroxycinnamaldéhyde, le 4-hydroxycinnamaldéhyde, le 2,4-dihydroxycinnamaldéhyde, la 4-hydroxybenzylidène-acétone, la 4-hydroxy-3-méthoxybenzylidène-acétone, le 4-hydroxy-3-méthoxycinnamaldéhyde (coniférylaldéhyde), le 3,5-diméthoxy-4-hydroxycinnamaldéhyde, le 4-hydroxycinnamylidène-acétaldéhyde, le 4-méthoxycinnamylidène-alcétaldéhyde.

6. Agent selon la revendication 5, **caractérisé en ce que** le composé supplémentaire est choisi dans le groupe formé par la N-(2-hydroxyéthyl)-N-éthyl-, 2-chloro-p-phénylènediamine, la N,N-bis-(2-hydroxyéthyl)-p-phénylènediamine, le 4-aminophénol, la p-phénylènediamine, le 2-(2,5-diaminophényl)-éthanol, le 2,5-diaminotoluène, la 3,4-méthylènedioxyaniline, le 2-amino-4-(2-hydroxyéthylamino)-anisole, le 2-(2,4-diaminophénoxy)-éthanol, le 3-amino-2,4-dichloro-, 2-méthyl-5-amino-, 3-méthyl-4-amino-, 2-méthyl-5-(2-hydroxyéthylamino)-, 2-méthyl-5-amino-4-chloro-, 6-méthyl-3-amino-2-chloro-, 2-aminométhyl-4-aminophénol, le 2-diéthylaminométhyl-4-aminophénol, le 2-diméthylaminométhyl-4-aminophénol, le 2,6-dichloro-4-aminophénol, le 2-hydroxyméthyl-4-aminophénol, le 3,4-méthylène-dioxyphénol, l'acide 3,4-diaminobenzoïque, la 2,5-diamino-, 2-diméthylamino-5-amino-, 3-amino-2-méthylamino-6-méthoxy-, 2,3-diamino-6-méthoxy-, 3,5-diamino-2,6-diméthoxy-, 2,6-dihydroxy-3,4-diméthylpyridine, la 2-hydroxy-4,5,6-triamino-, 4-hydroxy-2,5,6-triamino-, 2,4,5,6-tétraamino-, 2-méthylamino-4,5,6-triamino-pyrimidine, le 3,5-diaminopyrazole, le 3-amino-5-hydroxypyrazole, le 5,6-dihydroxyindole, et la 5,6-dihydroxyindoline, la β-alanine, la L-proline, la L-lysine, la DL-tyrosine, ainsi qu'à chaque fois les sels de ces composés, acceptables sur le plan physiologique, formés avec des acides de préférence inorganiques.

7. Agent selon l'une quelconque des revendications 1 à 6, **caractérisé en ce qu'**il contient des intensificateurs de couleur, choisis dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine, ou leurs mélangés quelconques.

8. Agent selon l'une quelconque des revendications 1 à 7, **caractérisé en ce qu'**il contient des colorants montant directement sur la fibre issus du groupe des nitrophénylènediamines, des nitroaminophénols, des anthraquinones, ou des indophénols, de préférence en une quantité de 0,01 à 20% en poids, par rapport à la teinture totale.

9. Agent selon l'une quelconque des revendications 1 à 8, **caractérisé en ce que** des sels d'ammonium ou métalliques choisis dans le groupe des formiates, carbonates, halogénures, sulfates, butyrates, valériates, caproates, acétates, lactates, glycolates, tartrates, citrates, gluconates, propionates, phosphates, et phosphonates, de métaux alcalins, comme le potassium, le sodium, ou le lithium, de métaux alcalino-terreux comme le magnésium, le calcium, le strontium ou le baryum, ou d'aluminium, de manganèse, de fer, de cobalt, de cuivre, ou de zinc, sont ajoutés.

10. Agent selon l'une quelconque des revendications 1 à 9, **caractérisé en ce qu'**il contient les agents d'oxydation, en particulier H₂O₂, en une quantité de 0,01 à 6% en poids, par rapport à la solution d'application.

11. Agent selon l'une quelconque des revendications 1 à 10, **caractérisé en ce qu'**il contient des tensio-actifs anioniques, zwitterioniques, ou non ioniques.

12. Utilisation de dérivés cyclohexénone de formule I, 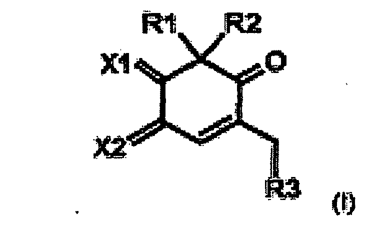 dans laquelle
R¹, R², R³ représentent indépendamment l'un de l'autre, un atome d'hydrogène, ou un groupe alkyle en C₁ à C₄, R¹ et R² pouvant former également ensemble un cycle, X¹ et X² représentent un atome d'oxygène, un atome de soufre ou conjointement un groupe 1,2-arylène-diimino, qui, de son côté peut être substitué dans le groupe aromatique par des atomes d'halogène, des groupes alkyle en C₁ à C₄, alcoxy en C₁ à C₄, nitro, amino, alkylamino en C₁ à C₄, hydroxy, carboxy, sulfo, ou un noyau aromatique condensé supplémentaire, et peut être quaternisé sur un des atomes d'azote par un groupe alkyle C₁ à C₆, aralkyle, aryle, alcényle en C₂ à C₄, hydroxyalkyle en C₁ à C₆, ou carboxyalkyle
le noyau aromatique condensé supplémentaire pouvant être substitué par des atomes d'halogène, des groupes alkyle en C₁ à C₄, alcoxy en C₁ à C₄, nitro, amino, alkylamino en C₁ à C₄, hydroxy, carboxy, sulfo,
ou ses formes tautomères ou ses sels acceptables sur le plan physiologique sous la forme d'un composant teignant dans des teintures capillaires par oxydation.

13. Procédé pour teindre des fibres kératiniques, en particulier les cheveux humains, dans lequel une teinture, contenant
A) au moins un dérivé cyclohexénone de formule I 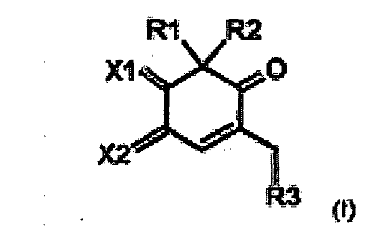 dans laquelle
R¹, R², R³ représentent indépendamment l'un de l'autre, un atome d'hydrogène, ou un groupe alkyle en C₁ à C₄, R¹ et R² pouvant former également ensemble un cycle,
X¹ et X² représentent un atome d'oxygène, un atome de soufre ou conjointement un groupe 1,2-arylène-diimino, qui, de son côté peut être substitué dans le groupe aromatique par des atomes d'halogène, des groupes alkyle en C₁ à C₄, alcoxy en C₁ à C₄, nitro, amino, alkylamino en C₁ à C₄, hydroxy, carboxy, sulfo, ou un noyau aromatique condensé supplémentaire, et peut être quaternisé sur un des atomes d'azote par un groupe alkyle C₁ à C₆, aralkyle, aryle, alcényle en C₂ à C₄, hydroxyalkyle en C₁ à C₆ ou carboxyalkyle,
le noyau aromatique condensé supplémentaire pouvant être substitué par des atomes d'halogène, des groupes alkyle en C₁ à C₄, alcoxy en C₁ à C₄, nitro, amino, alkylamino en C₁ à C₄, hydroxy, carboxy, sulfo,
ou ses formes tautomères ou ses sels acceptables sur le plan physiologique
B) au moins un composé choisi parmi les composés ayant un groupe amino primaire ou secondaire, ou un groupe hydroxy, choisi parmi les amines aromatiques primaires ou secondaires, les composés hétérocycliques azotés, et les composés hydroxyaromatiques, les acides aminés, les oligopeptides construits à partir de 2 à 9 acides aminés, et/ou les aldéhydes aromatiques,
ainsi que des ingrédients cosmétiques courants, est appliquée sur les fibres kératiniques, pendant quelque temps, couramment environ 30 minutes, laissée sur les fibres, et éliminée ensuite à nouveau par rinçage ou par lavage avec un shampoing.
